# EUROPEAN PATENT SPECIFICATION

(11) **EP 4 342 405 B1**
(45) Date of publication and mention of the grant of the patent: **20.08.2025**
(21) Application number: 23198180.4
(22) Date of filing: 19.09.2023
(51) Int. Cl.: A61B 18/14

(54) **CATHETER SHAFT WITH MULTI-PLANE ARTICULATION AND ROTATION**
KATHETERSCHAFT MIT MEHREBENENGELENK UND ROTATION
TIGE DE CATHÉTER AVEC ARTICULATION ET ROTATION MULTI-PLANS

(30) Priority: 20.09.2022 US 202263408276 P; 28.08.2023 US 202318457122
(43) Date of publication of application: 27.03.2024
(73) Proprietor: BIOSENSE WEBSTER (ISRAEL) LTD., 2066717 Yokneam (IL)
(72) Inventor: VAN NIEKERK, Pieter E., Irvine, 92618 (US)
(74) Representative: Carpmaels & Ransford LLP

(56) References cited:
- EP-A1- 3 881 755
- US-A1- 2013 158 379
- US-A1- 2020 405 182
- US-A1- 2021 085 386

## Description

### FIELD OF INVENTION

This invention relates to catheters with deflectable shafts, in particular, catheters with control handle actuators for manipulating deflectable shafts.

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by applying electrical energy, ablation fluid, or other some other mode of ablation, it may be possible to cease or modify the propagation of unwanted electrical signals from one portion of the heart to another. Ablating electrical energy may include radiofrequency (AC type) electrical energy, pulsed field (DC type) electrical energy (e.g., irreversible electroporation, etc.), or some other form of electrical energy. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with electrical energy to thereby ablate the contacted tissue. In some cases, the electrodes may be bipolar. In some other cases, a monopolar electrode may be used in conjunction with a ground pad or other reference electrode that is in contact with the patient. Irrigation may be used to draw heat from ablating components of an ablation catheter; and to prevent the formation of blood clots near the ablation site.

Examples of ablation catheters are described in U.S. Pub. No. 2013/0030426, entitled "Integrated Ablation System using Catheter with Multiple Irrigation Lumens," published January 31, 2013; U.S. Pub. No. 2017/0312022, entitled "Irrigated Balloon Catheter with Flexible Circuit Electrode Assembly," published November 2, 2017; U.S. Pub. No. 2018/0071017, entitled "Ablation Catheter with a Flexible Printed Circuit Board," published March 15, 2018; U.S. Pub. No. 2018/0056038, entitled "Catheter with Bipole Electrode Spacer and Related Methods," published March 1, 2018; U.S. Pat. No. 10,130,422, entitled "Catheter with Soft Distal Tip for Mapping and Ablating Tubular Region," issued November 20, 2018; U.S. Pat. No. 8,956,353, entitled "Electrode Irrigation Using Micro-Jets," issued February 17, 2015; and U.S. Pat. No. 9,801,585, entitled "Electrocardiogram Noise Reduction," issued October 31, 2017.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998**.** Examples of EP mapping catheters are described in U.S. Pat. No. 9,907,480, entitled "Catheter Spine Assembly with Closely-Spaced Bipole Microelectrodes," issued March 6, 2018; U.S. Pat. No. 10,130,422, entitled "Catheter with Soft Distal Tip for Mapping and Ablating Tubular Region," issued November 20, 2018; and U.S. Pub. No. 2018/0056038, entitled "Catheter with Bipole Electrode Spacer and Related Methods," published March 1, 2018.

Some catheter procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016; and various other references that are cited herein.

US 2021/085386 A1 teaches an apparatus including a handle, a catheter, and an end effector. The catheter extends distally from the handle. The catheter includes a body, a first cable, a second cable, and a third cable. The first cable is positioned in a first lumen of the catheter body and is operable to translate relative to the body of the catheter. The second cable is positioned in a second lumen of the catheter body and is operable to translate relative to the body of the catheter. The third cable is positioned in a third lumen of the catheter body and is operable to translate relative to the body of the catheter. The end effector extends distally from the catheter. The end effector includes at least one electrode.

The tissue of interest for diagnostic or treatment of organs including the heart may vary greatly. The tissue surface may be flat or undulating with caverns, and an indirect approach may be required to reach the tissue. Depending on the configuration of an end effector that may be equipped for mapping or ablation, a catheter shaft may need combinations of deflection, rotation and translation in order to accomplish tissue contact for successful diagnosis or treatment.

Accordingly, applicant recognized that there is a need to provide a catheter that provides that enables combinations of deflection, rotation and translation in response to manipulations of a control handle. While several catheter systems and methods have been made and used, it is believed that no one prior to the inventors has made or used the invention described, illustrated and claimed herein.

### SUMMARY OF THE INVENTION

The invention is defined in independent claim 1. Embodiments of the invention are described in the dependent claims.

### SUMMARY OF THE DISCLOSURE

In some embodiments, a catheter comprises an elongated flexible catheter body, an end effector, a first tensile member and a control handle. The catheter body defines a longitudinal axis and includes an inner shaft and an outer shaft. The end effector is affixed to a distal end of the outer shaft. The first tensile member extends along the longitudinal axis, with its distal end affixed to a distal portion of the inner shaft. The control handle is proximal of the catheter body and includes a first control member that is configured to pivot at a joint about an axis generally perpendicular to the longitudinal axis. A proximal end of the first tensile member is affixed to the first control member at a first location laterally offset from the joint such that the first tensile member deflects a distal portion of the inner shaft toward a first side of the control handle when the first control member pivots about the joint in a first direction.

In some embodiments, the catheter comprises a second tensile member that extends along the longitudinal axis, with a distal end affixed to the distal portion of the inner shaft. A proximal end of the second tensile member is affixed to the first control member at a second location laterally offset from the joint and opposite to the first location such that the second tensile member deflects the distal portion of the inner shaft toward a second side of the control handle opposite to the first side when the first control member pivots about the joint in a second direction opposite to the first direction.

In some embodiments, deflection of the distal portion of the inner shaft effects deflection of a distal portion of the outer shaft.

In some embodiments, the outer shaft is configured for rotation about the longitudinal axis relative to the inner shaft.

In some embodiments, the outer shaft is configured for translation along the longitudinal axis relative to the inner shaft.

In some embodiments, the catheter comprises an additional control member affixed to the outer shaft and configured to control rotation about the longitudinal axis of the outer shaft relative to the inner shaft.

In some embodiments, the catheter comprises an additional control member affixed to the outer shaft and configured to control translation along the longitudinal axis relative to the inner shaft.

In some embodiments, the catheter comprises an additional control member affixed to the other shaft and configured to control rotation about the longitudinal axis of the outer shaft and translation of the outer shaft relative to the inner shaft.

In some embodiments, the additional control member includes an indicator of a rotational or translational position of the outer shaft.

In some embodiments, the indicator includes a tactile indicator.

In some embodiments, the indicator includes a visual indicator.

In some embodiments, the additional control member is distal of the first control member.

In some embodiments, the inner shaft comprises an off-axis lumen and the first tensile member extends through the off-axis lumen.

In some embodiments, the inner shaft comprises at least first and second diametrically-opposing lumens, and the first tensile member extends through the first diametrically-opposed lumen and the second tensile member extends through the second diametrically-opposed lumen.

In some embodiments, at least one of the first and second tensile members extends between the inner and outer shafts.

In some embodiments, at least one of the first and second tensile member is embedded in a side wall of the inner shaft.

In some embodiments, the end effector includes a planar structure configured to support an array of electrodes.

In some embodiments, the rotation about the longitudinal axis of the outer shaft ranges between about 0 and 360 degrees.

In some embodiment, the rotation about the longitudinal axis of the outer shaft relative to the inner shaft ranges between about 0 and 90 degrees.

In some embodiment, the catheter shaft includes stop members configured to limit translation along the longitudinal axis of the outer shaft relative to the inner shaft to a predetermined range of distance.

In some embodiments, the stop members include a female member and a male member, the female member configured with proximal and distal stops defining a translating region therebetween with a predetermined length parallel with the longitudinal axis, the male member configured to translate in the translating region between the proximal and distal stops.

In some embodiments, one of the female and male members is disposed on one of the inner surface of the outer shaft and the outer surface of the inner shaft, and the the other of female and male members is disposed on the other of the inner surface of the outer shaft and the outer surface of the inner shaft.

In some embodiments, at least one of the female and male members extends circumferentially on one of the inner surface of the outer shaft and the outer surface of the inner shaft.

In some embodiments, the other of the female and male members extends radially from the other of the inner surface of the outer shaft and the outer surface of the inner shaft.

In some embodiments, the other of the female and male members extends circumferentially on the other of the inner surface of the other shaft and and the other surface of the inner shaft.

In some embodiments, the other of the female and male members is configured as a body with a radial dimension.

In some embodiments, the predetermined length of the translating region along the longitudinal axis ranges between about 0 and 1.0".

In some embodiments, an apparatus comprises a body, a catheter, an end effector, a first actuator and a second actuator. The catheter extends distally from the body, defining a longitudinal axis. The end effector us at a distal end of the catheter, defining an end effector plane. The first actuator is operable to rotate the end effector about the longitudinal axis between a first angular position and a second angular position and thereby re-orient the end effector plane between the first angular position and the second angular position. The second actuator is operable to deflect the end effector away from the longitudinal axis along a deflection plane that is parallel with the end effector plane when the end effector is in the first angular position about the longitudinal axis and to deflect the end effector away from the longitudinal axis along a deflection plane that is offset from the end effector plane when the end effector is in the second angular position about the longitudinal axis.

In some embodiments, the body including a grip portion configured to be grasped by a user.

In some embodiments, the first actuator being rotatably coupled with the body.

In some embodiments, the first actuator includes a knob, the knob being rotatable relative to the body about the longitudinal axis.

In some embodiments, the second actuator is rotatably coupled with the body.

In some embodiments, the second actuator includes a knob, the knob being rotatable relative to the body about a rotation axis perpendicular to the longitudinal axis.

In some embodiments, the catheter includes an inner shaft and an outer shaft.

In some embodiments, the end effector is secured to the outer shaft.

In some embodiments, the outer shaft is rotatable relative to the inner shaft, about the longitudinal axis.

In some embodiments, the first actuator being secured to a proximal end of the outer shaft.

In some embodiments, the second actuator is operable to drive deflection of a distal portion of the inner shaft away from the longitudinal axis, the inner shaft being operable to drive deflection of the outer shaft and the end effector away from the longitudinal axis in response to actuation of the second actuator.

In some embodiments, the catheter further comprises one or more articulation drive elements extending along the catheter, the one or more articulation drive elements coupling the second actuator with the distal portion of the inner shaft.

In some embodiments, the one or more articulation drive elements includes one or more drive cables.

In some embodiments, the end effector includes one or more electrodes.

In some embodiments, the one or more electrodes is configured to perform electrophysiology (EP) mapping.

In some embodiments, the end effector is operable to transition between a collapsed state and an expanded state.

In some embodiments, the end effector includes one or more position sensors configured to generate signals indicating a position of the end effector in three-dimensional space.

In some embodiments, the end effector is further operable to translate relative to the body along the longitudinal axis.

In some embodiments, the first actuator is operable to drive translation of the end effector relative to the body along the longitudinal axis.

In some embodiments, the first and second angular positions is angularly offset from each other by 90 degrees, the second actuator being operable to deflect the end effector away from the longitudinal axis along a deflection plane that is perpendicular to the end effector plane when the end effector is in the second angular position about the longitudinal axis.

In some embodiments, an apparatus, comprising a body, catheter, an end effector, a first actuator and a second actuator. The catheter extends distally from the body, defining a longitudinal axis, and includes an inner shaft, and an outer shaft, the outer shaft being rotatable relative to the body and relative to the inner shaft, about the longitudinal axis. The end effector is secured to a distal end of the outer shaft, the end effector being rotatable with the outer shaft relative to the body and relative to the inner shaft, about the longitudinal axis. The first actuator is operable to rotate the outer shaft relative to the body, about the longitudinal axis, and thereby rotate the end effector relative to the body, about the longitudinal axis. The second actuator is operable to deflect a distal portion of the inner shaft away from the longitudinal axis, the distal portion of the inner shaft being operable to drive a distal portion of the outer shaft away from the longitudinal axis and thereby deflect the end effector away from the longitudinal axis.

In some embodiments, the end effector defining an end effector plane, the end effector plane being defined such that the end effector plane will rotate about the longitudinal axis as the end effector is rotated relative to the body about the longitudinal axis.

In some embodiments, the first actuator is operable to rotate the end effector about the longitudinal axis between a first angular position and a second angular position, and the second actuator is operable to deflect the end effector away from the longitudinal axis along a deflection plane that is parallel with the end effector plane when the end effector is in the first angular position about the longitudinal axis, the second actuator being operable to deflect the end effector away from the longitudinal axis along a deflection plane that is offset from the end effector plane when the end effector is in the second angular position about the longitudinal axis.

In some embodiments, the outer shaft is operable to translate longitudinally relative to the body and relative to the inner shaft, along the longitudinal axis, the end effector being operable to translate longitudinally with the outer shaft relative to the body and relative to the inner shaft, along the longitudinal axis.

In some embodiments, an apparatus, comprising a body, a catheter, an end effector, a first actuator and a second actuator. The catheter extends distally from the body, the catheter defining a longitudinal axis and includes an inner shaft, and an outer shaft, the outer shaft being operable to translate longitudinally relative to the body and relative to the inner shaft, along the longitudinal axis. The end effector secured to a distal end of the outer shaft, the end effector being operable to translate longitudinally with the outer shaft relative to the body and relative to the inner shaft, along the longitudinal axis. The first actuator is operable to translate the outer shaft relative to the body, along the longitudinal axis, and thereby translate the end effector relative to the body, along the longitudinal axis. The second actuator is operable to deflect a distal portion of the inner shaft away from the longitudinal axis, the distal portion of the inner shaft being operable to drive a distal portion of the outer shaft away from the longitudinal axis and thereby deflect the end effector away from the longitudinal axis.

In some embodiments, the outer shaft is rotatable relative to the body and relative to the inner shaft, about the longitudinal axis, the end effector being rotatable with the outer shaft relative to the body and relative to the inner shaft, about the longitudinal axis.

In some embodiments, the end effector defines an end effector plane, the first actuator being operable to rotate the end effector about the longitudinal axis between a first angular position and a second angular position and thereby rotate the end effector plane about the longitudinal axis between the first angular position and the second angular position, and the second actuator is operable to deflect the end effector away from the longitudinal axis along a deflection plane that is parallel with the end effector plane when the end effector is in the first angular position about the longitudinal axis, the second actuator being operable to deflect the end effector away from the longitudinal axis along a deflection plane that is offset from the end effector plane when the end effector is in the second angular position about the longitudinal axis.

### BRIEF DESCRIPTION OF THE DRAWINGS

The drawings and detailed description that follow are intended to be merely illustrative and are not intended to limit the scope of the invention as contemplated by the inventors. These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1 depicts a schematic view of a medical procedure in which a catheter of a catheter assembly is inserted in a patient;
FIGS. 2A and 2B depict a schematic view of proximal and distal portions, respectively, of the catheter assembly of FIG. 1, with the catheter assembly in a first state of operation, and with the distal portion of the catheter assembly shown in partial cross-section;
FIGS. 3A and 3B depict a schematic view of proximal and distal portions, respectively, of the catheter assembly of FIG. 1, with the catheter assembly in a second state of operation, and with the distal portion of the catheter assembly shown in partial cross-section;
FIGS. 4A and 4B depict a schematic view of proximal and distal portions, respectively, of the catheter assembly of FIG. 1, with the catheter assembly in a third state of operation, and with the distal portion of the catheter assembly shown in partial cross-section;
FIG. 5 depicts an end cross-sectional view of the catheter body of FIG. 1, including an inner shaft, an outer shaft, and first and second tensile members, taken across line A-A, according to a first embodiment;
FIG. 6 depicts an end cross-sectional view of the catheter body of FIG. 1, including an inner shaft, an outer shaft, and first and second tensile members, taken across line A-A, according to a second embodiment;
FIG. 7A depicts a side cross-sectional view of the catheter body of FIG. 1, including an inner shaft, an outer shaft, and stop members, according to a first embodiment;
FIG. 7B depicts an end cross-sectional view of the catheter body of FIG. 7A, taken along line B-B;
FIG. 8A depicts a side cross-sectional view of the catheter body of FIG. 1, including an inner shaft, an outer shaft, and stop members, according to a second embodiment; and
FIG. 8B depicts an end cross-sectional view of the catheter body of FIG. 8A, taken along line B-B.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 70% to 110%. In addition, as used herein, the terms "patient," "host," "user," and "subject" refer to any human or animal subject and are not intended to limit the systems or methods to human use, although use of the subject invention in a human patient represents a preferred embodiment.

### I. Overview

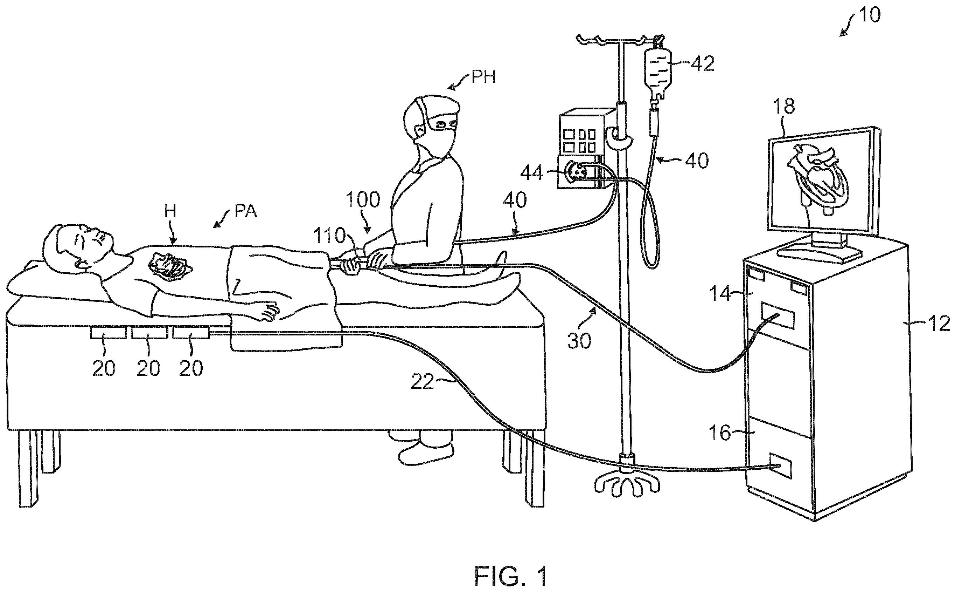

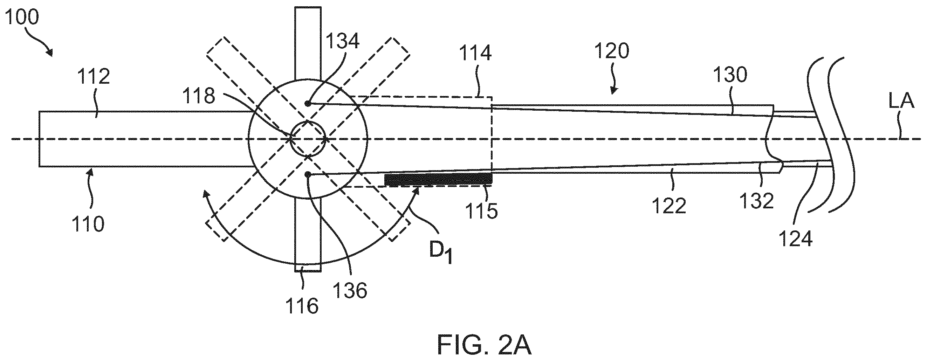

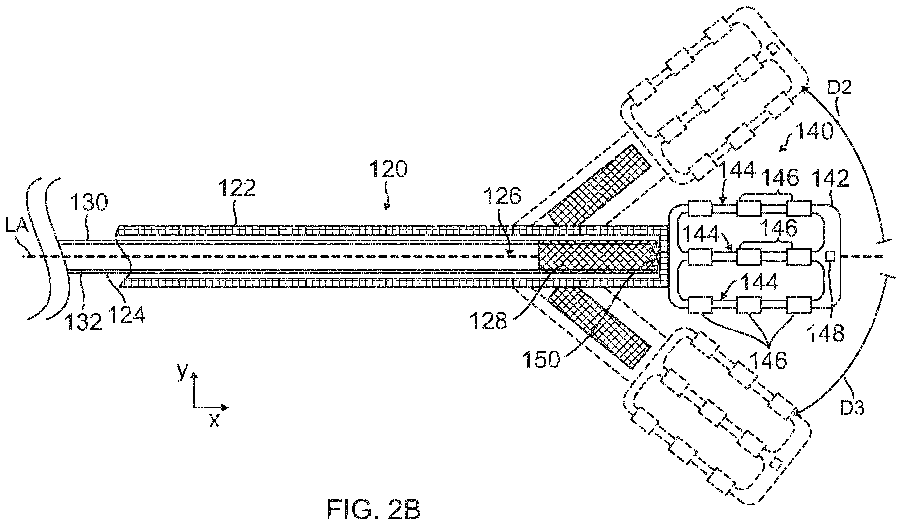

FIG. 1 shows an exemplary medical procedure and associated components of a cardiac catheter system that may be used to provide EP mapping or cardiac ablation as referred to above. In particular, FIG. 1 shows a physician (PH) grasping a handle (110) of a catheter assembly (100), with an end effector (140) of a catheter (120) (shown in FIGS. 2-4 but not shown in FIG. 1) of catheter assembly (100) disposed in a patient (PA) to map potentials in tissue and/or ablate tissue in or near the heart (H) of the patient (PA). As shown in FIGS. 2-4, catheter assembly (100) includes handle (110), catheter (120) extending distally from handle (110), and an end effector (140) located at a distal end of catheter (120). As will be described in greater detail below, end effector (140) may include various components configured to deliver electrical energy to targeted tissue sites, provide EP mapping functionality, track external forces imparted on end effector (140), track the location of end effector (140), and/or disperse fluid.

As shown in FIGS. 2-4, catheter (120) includes an elongate outer shaft (122) and an elongated inner shaft (124). Shafts (122, 124) are flexible. Outer shaft (122) is operable to rotate and translate relative to inner shaft (124), as will be described in greater detail below. End effector (140) is disposed at the distal end of outer shaft (122). End effector (140) and other various features and functionalities of catheter (120) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Magnetic field generators (20) are optional and may be omitted in some versions.

As shown in FIG. 1, guidance and drive system (10) of the present example include a console (12) and a display (18). Console (12) includes a first driver module (14) and a second driver module (16). First driver module (14) is coupled with catheter assembly (100) via cable (30). First driver module (14) of the present example is operable to receive EP mapping signals obtained via sensing electrodes (146) of end effector (140) as described in greater detail below. Console (12) includes a processor (not shown) that processes such EP mapping signals and thereby provides EP mapping as is known in the art.

In some variations, first driver module (14) may be further operable to provide electrical power to a distal tip member (142) of end effector (140) to thereby ablate tissue. Second driver module (16) is coupled with magnetic field generators (20) via cable (22). Second driver module (16) is operable to activate magnetic field generators (20) to generate an alternating magnetic field around the heart (H) of the patient (PA). For instance, field generators (20) may include coils that generate alternating magnetic fields in a predetermined working volume that contains the heart (H).

First driver module (14) may also be operable to receive position indicative signals from a position sensor assembly (148) in or near end effector (140), which will be described in greater detail below. The processor of console (12) is operable to process the position indicative signals from position sensor assembly (148) to thereby determine the position of end effector (140) within the patient (PA). Other components and techniques that may be used to generate real-time position data associated with end effector (140) may include wireless triangulation, acoustic tracking, optical tracking, inertial tracking, and the like. Alternatively, end effector (140) may lack position sensor assembly (148).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from position sensor assembly (148) of end effector (140). For instance, as end effector (140) of catheter (120) moves within the patient (PA), the corresponding position data from position sensor assembly (148) may cause the processor of console (12) to update the patient anatomy views in display (18) in real time to depict the regions of patient anatomy around end effector (140) as end effector (140) moves within the patient (PA). Moreover, the processor of console (12) may drive display (18) to show locations of aberrant conductive tissue sites, as detected via EP mapping with end effector (140) or as otherwise detected. By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (140) on the images of the patient's anatomy, such as by superimposing an illuminated dot, a crosshair, a graphical representation of end effector (140), or some other form of visual indication. Such a superimposed visual indication may also move within the images of the patient anatomy on display (18) in real time as the physician moves end effector (140) within the patient (PA), thereby providing real-time visual feedback to the operator about the position of end effector (140) within the patient (PA) as end effector (140) moves within the patient (PA). The images provided through display (18) may thus effectively provide a video tracking the position of end effector (140) within a patient (PA), without necessarily having any optical instrumentation (i.e., cameras) viewing end effector (140). In the same view, display (18) may simultaneously visually indicate the locations of aberrant conductive tissue sites detected through EP mapping. The physician (PH) may thus view display (18) to observe the real time positioning of end effector (140) in relation to the mapped aberrant conductive tissue sites and in relation to images of the adjacent anatomical structures in the patient (PA).

Fluid source (42) of the present example includes a bag containing saline or some other suitable fluid. Conduit (40) includes a flexible tube that is further coupled with a pump (44), which is operable to selectively drive fluid from fluid source (42) to catheter assembly (100). In some variations, such fluid may be expelled through openings (not shown) of end effector (140). Some versions of end effector (140) may lack such openings, such that fluid source (42) and conduit (40) may be omitted in some versions.

As noted above, end effector (140) may include various components configured to deliver electrical energy to targeted tissue sites, provide EP mapping functionality, track external forces imparted on end effector (140), track the location of end effector (140) within the patient (PA), and/or disperse fluid. As shown in FIGS. 2-4, end effector (140) of the present example includes a body (142) defining a plurality of spines (144). Spines (144) extend along respective axes that are parallel to the longitudinal axis (LA) of catheter (120). Each spine (144) has a plurality of electrodes (146). In the present example, electrodes (146) are operable to pick up EP mapping signals in or near the heart (H) of the patient (PA) as is known in the art. While not shown, some versions of outer shaft (122) may also include one or more reference electrodes that may be utilized to pick up reference potentials from blood during an EP mapping procedure while electrodes (146) contact tissue. Such reference potentials may be used to reduce noise or far field signals, as is known in the art.

In some versions, electrodes (146) (or one or more additional electrodes) are operable to apply electrical energy to ablate tissue, such as radiofrequency (AC type) electrical energy, pulsed field (DC type) electrical energy (e.g., irreversible electroporation, etc.), or some other form of electrical energy. Such ablation functionality may be provided in addition to, or in lieu of, EP mapping functionality.

As also shown in FIGS. 2-4, end effector (140) of the present example includes a position sensor assembly (148). Position sensor assembly (148) may include one or more coils (not shown) that are operable to generate signals that are indicative of the position and orientation of end effector (140) within the patient (PA). The coils are configured to generate electrical signals in response to the presence of an alternating electromagnetic field generated by magnetic field generators (20). In some versions, position sensor assembly (148) is in the form of one or more flex circuits integrated on or in end effector (140). In addition to, or as an alternative to, position sensor assembly (148) being incorporated into or onto end effector (140), outer shaft (122) and/or inner shaft (124) may include a position sensor assembly. By way of further example only, alternative position sensing may include impedance measurement-based position sensing. Examples of such position sensing are described in U.S. Pat. No. 7,869,865, entitled "Current-Based Position Sensing," issued January 11, 2011, and U.S. Pat. No. 8,456,182, entitled "Current Localization Tracker," issued June 4, 2013. Alternatively, any other suitable position sensing technologies and techniques may be used.

Body (142) of the present example is resiliently biased to assume an expanded shape as shown in FIG. 2. However, body (142) also has sufficient flexibility to collapse inwardly, to thereby fit in a constraining guide sheath to facilitate transport of end effector (140) from a patient entry point toward a target location in the cardiovascular system. By way of example only, body (142) may include a resilient substrate, one or more resilient elements (e.g., nitinol elements, etc.) secured to a substrate and/or other features to resiliently bias body (142) toward the expanded shape shown in FIG. 2. In some other versions, end effector (140) includes a balloon that is inflatable to transition from a collapsed state to an expanded state. As yet another variation, end effector (140) may include one or more mechanical linkages or other features that are operable to drive end effector (140) to transition from a collapsed state to an expanded state. End effector (140) of the present example has a substantially flat, planar shape when in the expanded state. However, other variations of end effector (140) may have other kinds of configurations when in the expanded state. By way of example only, such alternative configurations may include, but are not limited to, substantially spherical shapes, bulbous shapes, basket shapes, spiral shapes, etc.

As noted above, first driver module (14) of console (12) is coupled with catheter assembly (100) via cable (30), such that first driver module (14) of the present example is operable to receive EP mapping signals from sensing electrodes (146) via cable (30). First driver module (14) of console (12) is also operable to receive position indicative signals from position sensor assembly (148) via cable (30). In versions where end effector (140) is operable to apply electrical energy to tissue, such electrical energy may be supplied via cable (30). Various kinds of electrical communication features may be utilized to provide electrical communication between end effector (140) and cable (30). By way of example only, one or more wires may extend along the length of catheter (120). In some versions, such wires are integrated into or onto outer shaft (122). In some other versions, such wires are interposed between shafts (122, 124). In still other versions, such wires are integrated into or onto inner shaft (124). As another variation, one or more flex circuits may be provided with traces that are used to provide paths of electrical communication from cable (30) to end effector (140). Such flex circuits may be positioned in any of the locations described above in the context of wires. Still other variations may include a combination of flex circuits and wires.

### II. Examples of Catheter Steering, Rotation, and Advancement Features

End effector (140) may be positioned in various locations within the cardiovascular system of the patient (PA). By way of example only, end effector (140) may be positioned within a chamber of the heart (H), within the pulmonary vein or some other tubular anatomical structure in or adjacent to the heart (H), or elsewhere. It may be desirable for catheter assembly (100) to include features that facilitate maneuvering of end effector (140) to such different kinds of anatomical structures. Moreover, the desirable kind of movement of end effector (140) may vary based on the kind of anatomical structure in which end effector (140) is disposed. It may therefore be desirable for catheter assembly (100) to include features that facilitate different kinds of movement of end effector (140) within different kinds of anatomical structures. Examples of such features will be described in greater detail below.

As noted above, catheter assembly (100) includes a handle (110). Handle (110) includes a proximal grip (112), a distal knob (114), and an articulation knob (116). Proximal grip (112) is configured for grasping by a hand of the physician (PH), though it is contemplated that some variations of catheter assembly (100) may be robotically controlled, such that not all implementations must be directly grasped by the hand of a human operator. Distal knob (114) is operable to rotate relative to proximal grip (112), about the longitudinal axis (LA). Distal knob (114) is also operable to translate longitudinally relative to proximal grip (112), along the longitudinal axis (LA). Articulation knob (116) is pivotably coupled with proximal grip (112) at a joint (118). Articulation knob (116) is operable to pivot at joint (118) along the x-y plane, in a pivot direction (D₁), about a pivot axis that is perpendicular to the longitudinal axis (LA).

A pair of articulation tensile members or cables (130, 132) couple articulation knob (116) with inner shaft (124) in this example. The proximal ends of articulation cables (130, 132) are secured to articulation knob (116) at respective anchor points (134, 136). Anchor points (134, 136) are laterally offset from pivot joint (118), at opposite sides of pivot point (118). Thus, as articulation knob (116) is rotated at pivot joint (118), such movement of articulation knob (116) and anchor points (134, 136) will cause articulation cables (130, 132) to simultaneously translate longitudinally in opposite directions. By way of further example only, the coupling and operability of articulation cables (130, 132) and articulation knob (116) may be provided in accordance with at least some of the teachings of U.S. Pub. No. 2020/0405182, entitled "Catheter Deflection System with Load Limiter," published December 31, 2020.

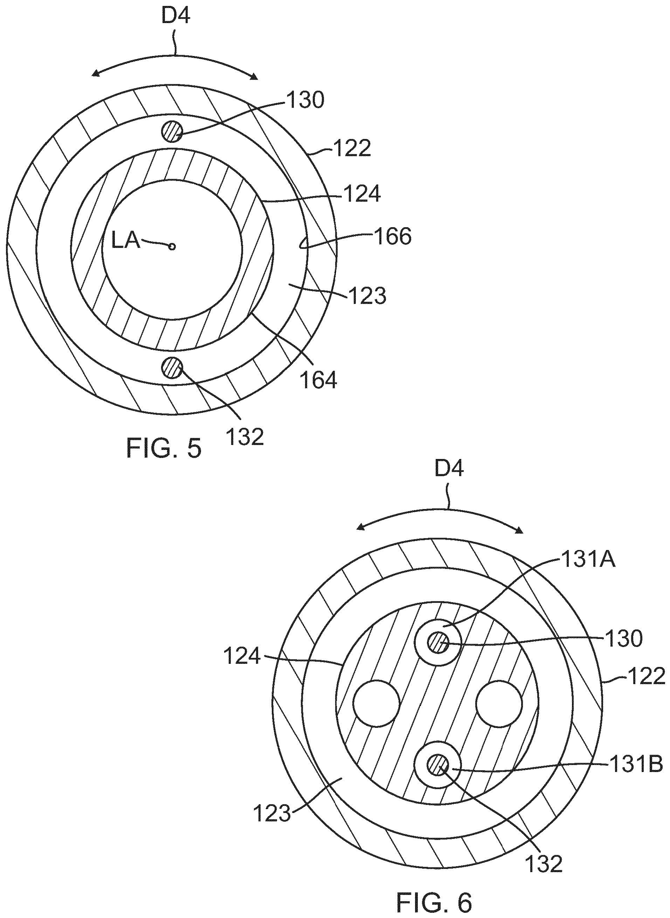

The distal ends of articulation cables (130, 132) are secured to the distal end (128) of inner shaft (128). Inner shaft (124) includes a laterally flexing region (126) just proximal to distal end (128). Thus, when articulation knob (116) is actuated to drive articulation cables (130, 132) to simultaneously translate longitudinally in opposite directions, such movement of articulation cables (130, 132) will cause inner shaft (124) to deflect laterally away from, or toward, the longitudinal axis (LA), at flexing region (126). As inner shaft (124) deflects laterally away from, or toward, the longitudinal axis (LA), at flexing region (126), inner shaft (124) urges outer shaft (122), or at least a portion thereof, to also deflect laterally away from, or toward, the longitudinal axis (LA). Since end effector (140) is fixedly secured to outer shaft (120) in this example, the lateral deflecting movement of outer shaft (122) will cause end effector (140) to also deflect laterally away from, or toward, the longitudinal axis (LA). Such movement is indicated in the different deflection directions (D₂, D₃) in FIG. 2. While articulation cables (130, 132) are used in the present example, other variations may use bands and/or other kinds of features to drive the lateral deflection movement of end effector (140). It should also be understood that articulation cables (130, 132) may be interposed between shafts (122, 124), as shown in FIG. 5, may extend along respective lumens, e.g., diametrically-opposed lumens 131A, 131B, formed in inner shaft (124), as shown in FIG. 6, or may be otherwise positioned.

In the operational state shown in FIG. 2, the directions (D₂, D₃) of lateral deflection movement of end effector (140) are along the x-y plane. In addition, the version of end effector (140) depicted in FIG. 2 is substantially planar; and the plane defined by end effector (140) is also along the x-y plane. Thus, in the operational state shown in FIG. 2, end effector (140) laterally deflects along the same plane defined by end effector (140) when articulation knob (116) is actuated.

In some scenarios, it may be desirable to provide lateral deflection of end effector (140) along the same plane defined by end effector (140) when articulation knob (116) is actuated as shown in FIG. 2. For instance, such movement may facilitate positioning of end effector (140) at a targeted tissue site. In addition, or in the alternative, such movement may facilitate a desired interaction between electrodes (146) and tissue at the target site. By way of example only, when catheter assembly (100) is in the operational state shown in FIG. 2, where end effector (140) will articulate along the same x-y plane along which end effector (140) extends, end effector (140) may have a desired movement along substantially flat tissue (e.g., in a chamber of the heart (H)). In this context, when end effector (140) is placed against the substantially flat tissue (e.g., along a tissue plane that also extends along the x-y plane), the operator may repeatedly actuate articulation knob (116) back and forth to effectively wipe end effector (140) along the plane of engagement between end effector (140) and tissue. This may facilitate or enhance contact between electrodes (146) and various regions of substantially tissue. Alternatively, there may be other scenarios where it is desirable to provide lateral deflection of end effector (140) along the same plane defined by end effector (140) when articulation knob (116) is actuated as shown in FIG. 2.

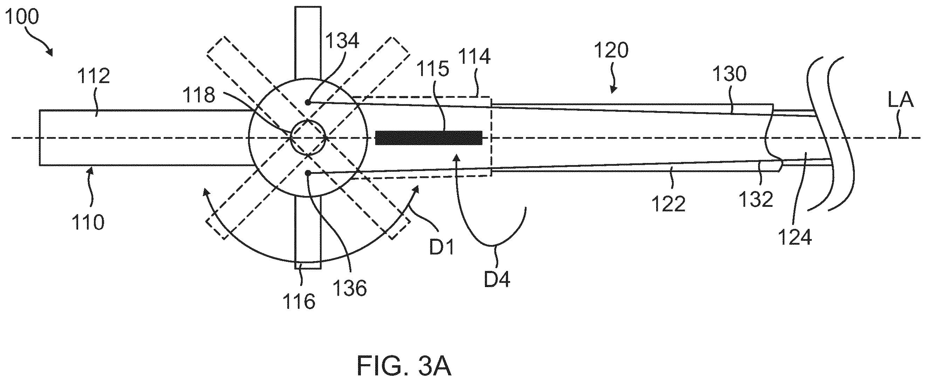

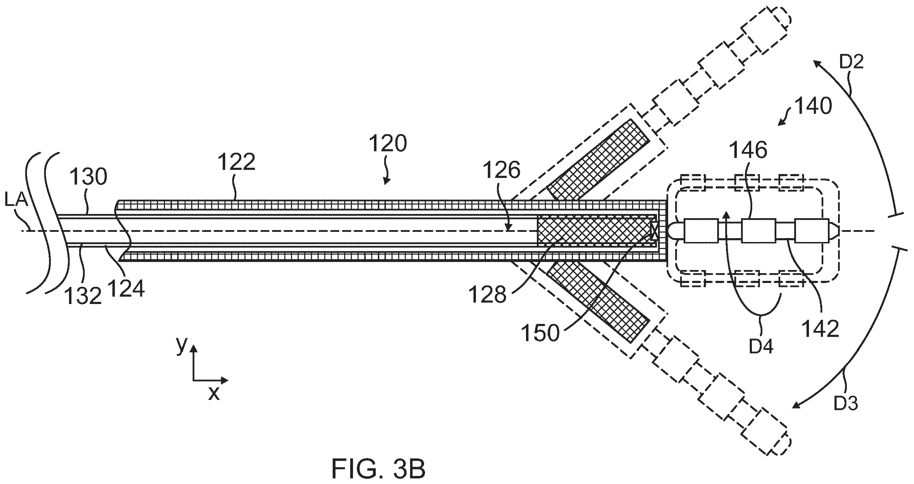

As noted above, distal knob (114) is operable to rotate relative to proximal grip (112), about the longitudinal axis (LA); and to translate longitudinally relative to proximal grip (112), along the longitudinal axis (LA). The proximal end of outer shaft (122) is secured to distal knob (114), while the distal end of outer shaft (122) is secured to end effector (140). Thus, when distal knob (114) is rotated relative to proximal grip (112), about the longitudinal axis (LA), outer shaft (122) and end effector (140) will rotate with distal knob (114), about the longitudinal axis (LA). Such rotational movement of distal knob (114), outer shaft (122), and end effector (140) is depicted in FIG. 3 as being along a rotational direction (D₄). While FIG. 3 primarily shows rotational movement of end effector (140) about the longitudinal axis (LA) while the distal portion of catheter (120) is in a straight configuration, end effector (140) may also be rotated in the rotational direction (D₄) while catheter (120) is in a laterally bent or articulated state. Regardless of whether end effector (140) is rotated in the rotational direction (D₄) while catheter (120) is in a straight configuration or a laterally bent/ articulated configuration, end effector (140) may be rotated in the rotational direction (D₄) while end effector (140) contacts tissue. Such spinning movement of end effector (140) may be particularly desirable in scenarios where end effector (140) is disposed in a tubular anatomical structure (e.g., a pulmonary vein). This spinning movement of end effector (140) may facilitate or enhance contact between electrodes (146) and various regions of tissue in a tubular anatomical structure.

Distal knob (114) may also be actuated to rotate end effector (140) about the longitudinal axis (LA) in order to effectively change the articulation mode provided by articulation knob (116). In particular, if distal knob (114) is rotated 90 degrees as shown in the transition from the state depicted in FIG. 2 to the state depicted in FIG. 3, the resulting rotation of end effector (140) will place the plane defined by end effector (140) along the x-z plane instead of the x-y plane. With end effector (140) being oriented along the x-z plane, actuation of articulation knob (116) will still articulate end effector (140) along the x-y plane. Thus, in the state shown in FIG. 3, end effector (140) will no longer articulate along the plane defined by end effector (140). Instead, end effector (140) will articulate along a plane (i.e., the x-z plane) that is perpendicular to the plane (i.e., the x-y plane) defined by end effector (140). It may be desirable to have end effector (140) articulate along this perpendicular plane to facilitate positioning of end effector (140) at a targeted site and/or for other purposes.

Distal knob (114) of the present example includes an angular orientation feature (115) that is configured to provide feedback to the physician (PH) on the angular orientation of end effector (140) about the longitudinal axis (LA). By way of example only, angular orientation feature (115) may include a stripe, a rib, or some other kind of indicia that provides visual and/or tactile feedback indicating the angular orientation of end effector (140) about the longitudinal axis (LA). In addition to including angular orientation feature (115), handle (110) may include detents, visual indicia, and/or other features that provide feedback to the physician (PH) indicating different 90 degree rotational positions of end effector (140) about the longitudinal axis (LA). For instance, such detents, visual indicia, etc., may be configured to indicate when end effector (140) is in the angular position about longitudinal axis (LA) as shown in FIG. 2 (i.e., when end effector (140) is along the same x-y plane of articulation); and when end effector (140) is in the angular position about longitudinal axis (LA) as shown in FIG. 3. (i.e., when end effector (140) is along an x-z plane that is perpendicular to the x-y plane of articulation). Some versions may only allow distal knob (114) to rotate 90 degrees about the longitudinal axis (LA), such that distal knob (114) may only rotate between the position shown in FIG. 2 and the position shown in FIG. 3. Other versions may allow distal knob (114) to rotate a full 360 degrees about the longitudinal axis (LA), more than 360 degrees about the longitudinal axis (LA) (e.g., infinitely), or to any other suitable extent.

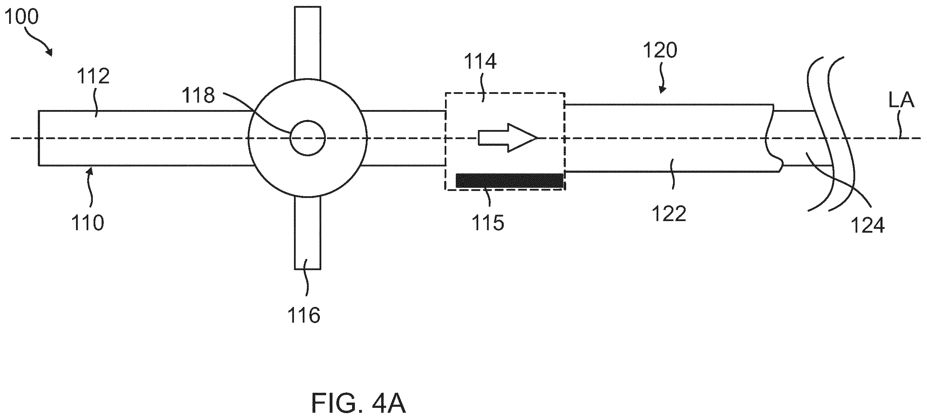

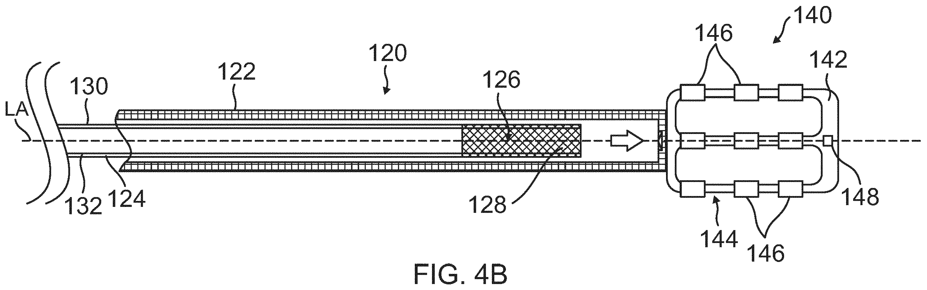

There may also be scenarios where it is desirable to increase the effective length of catheter (120), to thereby facilitate end effector (140) reaching a targeted tissue site. To that end, distal knob (114) of the present example is configured to advance relative to proximal grip (112), along the longitudinal axis (LA), from the position shown in FIGS. 2-3 to the position shown in FIG. 4. Since outer shaft (122) and end effector (140) are secured to distal knob (114), outer shaft (122) and end effector (140) will translate with distal knob (114), relative to proximal grip (112), along the longitudinal axis (LA). The resulting distal movement of end effector (140) will increase the distance between end effector (140) and proximal grip (140), thereby increasing the effective length of catheter (120). While FIG. 4 shows longitudinal movement of end effector (140) relative to proximal grip (112), along the longitudinal axis (LA), while the distal portion of catheter (120) is in a straight configuration, end effector (140) may also be translated relative to proximal grip (112), along the longitudinal axis (LA), while catheter (120) is in a laterally bent or articulated state. In either scenario, such translation of end effector relative to proximal grip (112), along the longitudinal axis (LA), may facilitate access for end effector (140) to reach a targeted tissue site. Moreover, the physician (PH) may repeatedly reciprocate distal knob (114) relative to proximal grip (112) to thereby slidingly reciprocate electrodes (146) along tissue. Regardless of how or when distal knob (114) is translated longitudinally relative to proximal grip (112), some versions of handle (110) may include one or more features that restrict such longitudinal movement to a certain range of movement (e.g., approximately 1 inch, etc.).

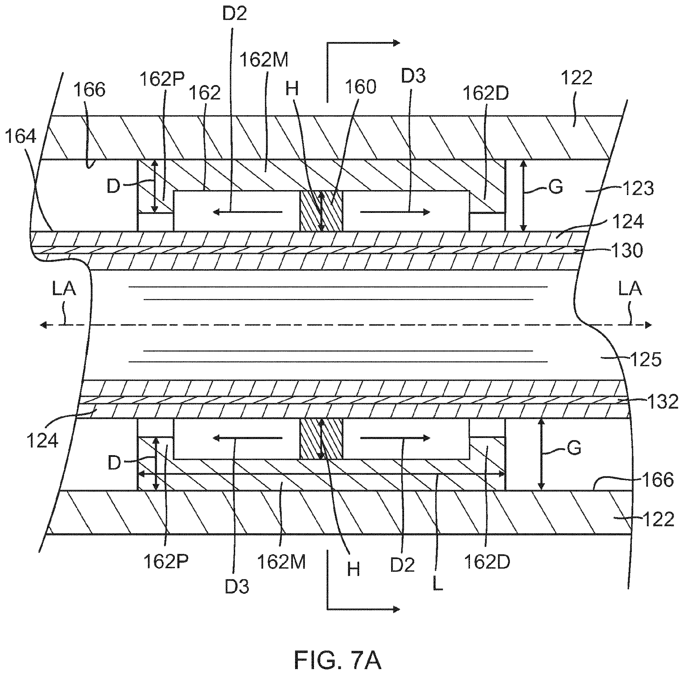

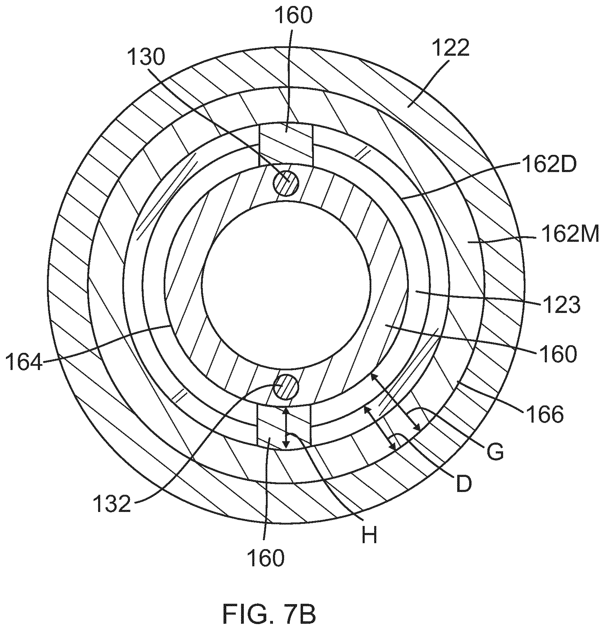

The catheter assembly may include stop members that are disposed between the outer shaft 122 and the inner shaft 124 (extending through a lumen 123 of the outer shaft 122) to restrict longitudinal movement of the outer shaft relative to the inner shaft 124 a predetermined range of distance L. In the illustrated embodiment of FIG. 7A and FIG. 7B, a first or male stop member 160 is disposed on outer surface 164 of the inner shaft 124 and a second or female stop member 162 is disposed on inner surface of the outer shaft 122. In some embodiments, the female member 162 has a circumferential body, e.g., a cylindrical member, that extends 360 degrees about the longitudinal axis LA and includes a proximal stop 162P, e.g., a proximal ring, and a distal stop 162D, e.g., a distal ring, that are separated by the distance L, where each stop has a radial dimension D that extends inwardly inside the lumen 123 of the outer shaft 122, from the inner surface 166 of the outer shaft 122 toward the outer surface 164 of the inner shaft 124. Configured as a discrete radial projection, one or more male members 160 extend outwardly toward the female member 162, situated between the proximal and distal stops 162P, 162D.

As the outer shaft 122 moves longitudinally relative to the inner shaft 124 between a retracted position or an extended position, the female member 162 moves relative to the male member 160 within the lumen 123 of the outer shaft 122 but its movement (and hence also movement of the outer shaft 122) is limited proximally and distally by the stops 162P, 162D, respectively, when each of these stops, respectively, abuts with the male member 160. In that regard, the male member 160 has a radial dimension H relative to the radial dimension D of the proximal and distal stops 162P,162D such that the stops 162P, 162D can abut with male member 160 in restricting the translation of the outer shaft 122 to the length L of the female member 162. The exact dimensions of D and H are not critical but a total of their combined dimensions (D+H) is to exceed a space gap G between the inner surface 166 of the outer shaft 122 and the outer surface 164 of the inner shaft 124 so that there is an overlap between the stops 162P, 162D and the male member 160.

To accommodate rotational movement of the outer shaft 122 relative to the inner shaft 124, it is understood at least one of the male and female members is configured with an extended body that spans 360 degrees circumferentially around its respective shaft, whereas the other of the male and female members may span similarly circumferentially or it may be configured as a discrete body with a radial projection or dimension.

It is further understood that in some embodiments the male member 160 may be disposed on the inner surface 166 of the outer shaft 122 and the female member 162 may be disposed on the outer surface 164 of the inner shaft 124.

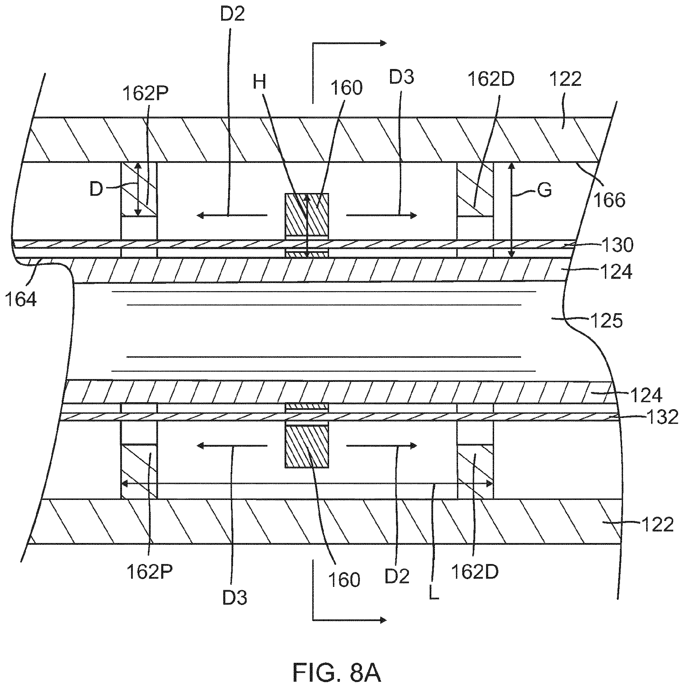

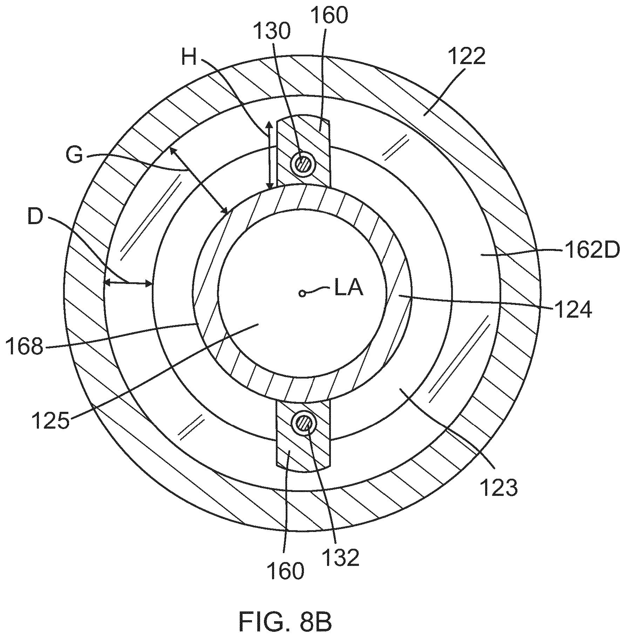

Although the female member 162 depicted in FIG. 7A and FIG. 7B includes a midportion 162M extending between the stops 162P, 162D, it is also understood that in some embodiments the female member 162 is without the midportion 162M and configured as discrete elements with a radial dimension, as shown in FIG. 8A and FIG. 8B. In any case, the combination of the proximal and distal stops 162P, 162D of the female member 162 serves as bi-directional stops abutting the male member 160 in restricting movement of the outer shaft 122 proximally and distally. However, it is also understood that in some embodiments the stop members consist of a pair of stops with one stop disposed on the inner surface 166 of the outer shaft 122, the other stop disposed on the outer surface 168 of the inner shaft 168. Depending on which stop is distal of the other stop, the pair of stops serve as unidirectional stops in restricting movement of the outer shaft 122 proximally or distally.

As noted above, one or more wires, conductive traces of a flex circuit, or other electrical communication conduits may extend along catheter (120) to electrically couple end effector (140) with cable (30). In versions where such electrical communication features are not positioned entirely on or in outer shaft (122), it may be desirable to configure such electrical communication features to accommodate the various kinds of movement of end effector (140) relative to handle (110) as described above. By way of example only, a slip coupling (150) may be provided at the proximal end of end effector

(140) to accommodate rotation of end effector (140) about the longitudinal axis (LA), while maintaining electrical continuity with electrical communication conduits (not shown), and without causing such electrical communication conduits to bind up in response to rotation of end effector (140) about the longitudinal axis (LA). In addition, such electrical communication conduits may include sliding contact features and/or service loop features that accommodate translation of end effector (140) along the longitudinal axis (LA) while maintaining electrical continuity during such translation of end effector (140) along the longitudinal axis (LA).

### III. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein.

Having shown and described various versions of the present invention, further adaptations of the methods and systems described herein may be accomplished by appropriate modifications by one skilled in the art. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention is defined by the following claims and is understood not to be limited to the details of structure and operation shown and described in the specification and drawings.

The preceding description has been presented with reference to presently preferred embodiments of the invention. Workers skilled in the art and technology to which this invention pertains will appreciate that alterations and changes in the described structure may be practised. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims.

## Claims

1. A catheter (120) comprising:
an elongated flexible catheter body defining a longitudinal axis (LA) and including an inner shaft (124) and an outer shaft (122);
an end effector (140) affixed to a distal end of the outer shaft;
a first tensile member (130) extending along the longitudinal axis, with its distal end affixed to a distal portion of the inner shaft; and
a control handle (110) proximal of the catheter body and including a first control member (116) that is configured to pivot at a joint (118) about an axis generally perpendicular to the longitudinal axis, a proximal end of the first tensile member affixed to the first control member at a first location laterally offset from the joint such that the first tensile member deflects a distal portion of the inner shaft toward a first side of the control handle when the first control member pivots about the joint in a first direction.

2. The catheter of claim 1, comprising a second tensile member (132) that extends along the longitudinal axis, with a distal end affixed to the distal portion of the inner shaft, a proximal end of the second tensile member affixed to the first control member at a second location laterally offset from the joint and opposite to the first location such that the second tensile member deflects the distal portion of the inner shaft toward a second side of the control handle opposite to the first side when the first control member pivots about the joint in a second direction opposite to the first direction, optionally wherein at least one of the first and second tensile members extends between the inner and outer shafts..

3. The catheter of claim 1, wherein deflection of the distal portion of the inner shaft urges deflection of a distal portion of the outer shaft.

4. The catheter of claim 1, wherein the outer shaft is configured for rotation about the longitudinal axis relative to the inner shaft, or for translation along the longitudinal axis relative to the inner shaft.

5. The catheter of claim 1, further comprising an additional control member (114) affixed to the outer shaft and configured to control (i) rotation about the longitudinal axis of the outer shaft relative to the inner shaft, (ii) translation along the longitudinal axis relative to the inner shaft, or (iii) control rotation about the longitudinal axis of the outer shaft and translation of the outer shaft relative to the inner shaft.

6. The catheter of claim 1, further comprising an indicator (115) of a rotational or translational position of the outer shaft, optionally wherein the indicator includes a tactile indicator or a visual indicator.

7. The catheter of claim 6, wherein the additional control member is distal of the first control member.

8. The catheter of claim 1, wherein the inner shaft comprises an off-axis lumen and the first tensile member extends through the off-axis lumen.

9. The catheter of claim 2, wherein the inner shaft comprises at least first and second diametrically-opposing lumens, and the first tensile member extends through the first diametrically-opposed lumen and the second tensile member extends through the second diametrically-opposed lumen.

10. The catheter of claim 1, the end effector includes a planar structure configured to support an array of electrodes.

11. The catheter of claim 1, wherein the rotation about the longitudinal axis of the outer shaft ranges between about 0 and 360 degrees, or the rotation about the longitudinal axis of the outer shaft relative to the inner shaft ranges between about 0 and 90 degrees.

12. The catheter of claim 1, further comprising stop members (160, 162) configured to limit translation along the longitudinal axis of the outer shaft relative to the inner shaft to a predetermined distance.

13. The catheter of claim 12, wherein the stop members include a female member and a male member, the female member configured with proximal and distal stops defining a translating region therebetween with a predetermined length parallel with the longitudinal axis, the male member configured to move in the translating region between the proximal and distal stops relative to the female member.

14. The catheter of claim 13, wherein one of the female and male members is disposed on one of the inner surface of the outer shaft and the outer surface of the inner shaft, and the other of female and male members is disposed on the other of the inner surface of the outer shaft and the outer surface of the inner shaft.

15. The catheter of claim 14, wherein at least one of the female and male members extends circumferentially on one of the inner surface of the outer shaft and the outer surface of the inner shaft.

16. The catheter of claim 15, wherein the other of the female and male members (i) extends from the other of the inner surface of the outer shaft and the outer surface of the inner shaft, (ii) extends circumferentially on the other of the inner surface of the other shaft and the other surface of the inner shaft, or (iii) is configured as a body with a radial dimension.

17. The catheter of claim 12, wherein the predetermined distance ranges between about 0 and 1.0".

## Patentansprüche

1. Katheter (120), umfassend:
einen länglichen flexiblen Katheterkörper, der eine Längsachse (LA) definiert und einen inneren Schaft (124) und einen äußeren Schaft (122) einschließt;
einen Endeffektor (140), der an einem distalen Ende des länglichen Schafts befestigt ist:
ein erstes ausziehbares Element (130), das sich entlang der Längsachse erstreckt, wobei dessen distales Ende an einem distalen Abschnitt des inneren Schafts befestigt ist; und
einen Steuergriff (110) proximal des Katheterkörpers und einschließlich eines ersten Steuerelements (116), das konfiguriert ist, um an einem Gelenk (118) um eine Achse, die im Allgemeinen zu der Längsachse senkrecht ist, zu schwenken, wobei ein proximales Ende des ersten ausziehbaren Elements an einer ersten Stelle seitlich von dem Gelenk versetzt an dem ersten Steuerelement befestigt ist, derart, dass das erste ausziehbare Element einen distalen Abschnitt des inneren Schafts zu einer ersten Seite des Steuergriffs lenkt, wenn das erste Steuerelement um das Gelenk in eine erste Richtung schwenkt.

2. Katheter nach Anspruch 1, umfassend ein zweites ausziehbares Element (132), das sich entlang der Längsachse erstreckt, wobei ein distales Ende an dem distalen Abschnitt des inneren Schafts befestigt ist, wobei ein proximales Ende des zweiten ausziehbaren Elements an einer zweiten Stelle seitlich von dem Gelenk versetzt und gegenüber der ersten Stelle an dem ersten Steuerelement befestigt ist, derart, dass das zweite ausziehbare Element den distalen Abschnitt des inneren Schafts zu einer zweiten Seite des Steuergriffs gegenüber der ersten Seite lenkt, wenn das erste Steuerelement um das Gelenk in eine zweite Richtung entgegengesetzt der ersten Richtung schwenkt, optional wobei sich mindestens eines von dem ersten und dem zweiten ausziehbaren Element zwischen dem inneren und dem äußeren Schaft erstreckt.

3. Katheter nach Anspruch 1, wobei eine Lenkung des distalen Abschnitts des inneren Schafts die Lenkung eines distalen Abschnitts des äußeren Schafts erzwingt.

4. Katheter nach Anspruch 1, wobei der äußere Schaft für eine Drehung um die Längsachse relativ zu dem inneren Schaft oder für eine Translation entlang der Längsachse relativ zu dem inneren Schaft konfiguriert ist.

5. Katheter nach Anspruch 1, ferner umfassend ein zusätzliches Steuerelement (114), das an dem äußeren Schaft befestigt ist und konfiguriert ist zum Steuern (i) der Drehung um die Längsachse des äußeren Schafts relativ zu dem inneren Schaft, (ii) der Translation entlang der Längsachse relativ zu dem inneren Schaft oder (iii) der Drehung um die Längsachse des äußeren Schafts und der Translation des äußeren Schafts relativ zu dem inneren Schaft.

6. Katheter nach Anspruch 1, ferner umfassend einen Indikator (115) für eine Dreh- oder Translationsposition des äußeren Schafts, optional wobei der Indikator einen taktilen Indikator oder einen visuellen Indikator einschließt.

7. Katheter nach Anspruch 6, wobei das zusätzliche Steuerelement distal von dem ersten Steuerelement ist.

8. Katheter nach Anspruch 1, wobei der innere Schaft ein außeraxiales Lumen umfasst und sich das erste ausziehbare Element durch das außeraxiale Lumen erstreckt.

9. Katheter nach Anspruch 2, wobei der innere Schaft mindestens ein erstes und ein zweites diametral gegenüberliegendes Lumen umfasst und sich das erste ausziehbare Element durch das erste diametral gegenüberliegende Lumen erstreckt und sich das zweite ausziehbare Element durch das zweite diametral gegenüberliegende Lumen erstreckt.

10. Katheter nach Anspruch 1, wobei der Endeffektor eine planare Struktur, die konfiguriert ist, um eine Elektrodenanordnung zu tragen, einschließt.

11. Katheter nach Anspruch 1, wobei die Drehung um die Längsachse des äußeren Schafts zwischen etwa 0 und 360 Grad liegt oder die Drehung um die Längsachse des äußeren Schafts relativ zu dem inneren Schaft zwischen etwa 0 und 90 Grad liegt.

12. Katheter nach Anspruch 1, ferner umfassend Anschlagelemente (160, 162), die konfiguriert sind, um die Translation entlang der Längsachse des äußeren Schafts relativ zu dem inneren Schaft auf eine zuvor bestimmte Distanz zu begrenzen.

13. Katheter nach Anspruch 12, wobei die Anschlagelemente ein Aufnahmeelement und ein Einführelement einschließen, wobei das Aufnahmeelement mit proximalen und distalen Anschlägen, die dazwischen einen Translationsbereich mit einer zuvor bestimmten Länge parallel zu der Längsachse definieren, konfiguriert ist, wobei das Einführelement konfiguriert ist, um sich in dem Translationsbereich zwischen den proximalen und distalen Anschlägen relativ zu dem Aufnahmeelement zu bewegen.

14. Katheter nach Anspruch 13, wobei eines von dem Aufnahmeelement und dem Einführelement auf einem von der inneren Oberfläche des äußeren Schafts und der äußeren Oberfläche des inneren Schafts angeordnet ist und das andere von dem Aufnahmeelement und dem Einführelement auf der anderen der inneren Oberfläche des äußeren Schafts und der äußeren Oberfläche des inneren Schafts angeordnet ist.

15. Katheter nach Anspruch 14, wobei sich mindestens eines von dem Aufnahmeelement und dem Einführelement in Umfangsrichtung auf der inneren Oberfläche des äußeren Schafts oder der äußeren Oberfläche des inneren Schafts erstreckt.

16. Katheter nach Anspruch 15, wobei das andere von dem Aufnahmeelement und dem Einführelement (i) sich von der anderen der inneren Oberfläche des äußeren Schafts und der äußeren Oberfläche des inneren Schafts erstreckt, (ii) sich in Umfangsrichtung auf der anderen der inneren Oberfläche des anderen Schafts und der anderen Oberfläche des inneren Schafts erstreckt oder (iii) als ein Körper mit einer radialen Abmessung konfiguriert ist.

17. Katheter nach Anspruch 12, wobei die zuvor bestimmte Distanz zwischen etwa 0 und 1,0" liegt.

## Revendications

1. Cathéter (120) comprenant :
un corps de cathéter flexible allongé définissant un axe longitudinal (LA) et comportant une tige interne (124) et une tige externe (122) ;
un effecteur (140) fixé fonctionnellement à une extrémité distale de la tige externe :
un premier élément de traction (130) s'étendant le long de l'axe longitudinal et dont l'extrémité distale est fixée à une partie distale de la tige interne ; et
une poignée de commande (110) proximale du corps de cathéter et comportant un premier élément de commande (116) conçue pour pivoter au niveau d'une articulation (118) autour d'un axe généralement perpendiculaire à l'axe longitudinal, une extrémité proximale du premier élément de traction fixée au premier élément de commande à un premier emplacement décalé latéralement par rapport à l'articulation de sorte que le premier élément de traction dévie une partie distale de la tige interne vers un premier côté de la poignée de commande lorsque le premier élément de commande pivote autour de l'articulation dans une première direction.

2. Cathéter selon la revendication 1, comprenant un second élément de traction (132) qui s'étend le long de l'axe longitudinal, avec une extrémité distale fixée à la partie distale de la tige interne, une extrémité proximale du second élément de traction fixée au premier élément de commande à un second emplacement latéralement décalé par rapport à l'articulation et opposé au premier emplacement, de sorte que le second élément de traction dévie la partie distale de la tige interne vers un second côté de la poignée de commande opposé au premier côté lorsque le premier élément de commande pivote autour de l'articulation dans une seconde direction opposée à la première direction, éventuellement dans lequel au moins parmi les premier et second éléments de traction s'étend entre les tiges interne et externe.

3. Cathéter selon la revendication 1, dans lequel la déviation de la partie distale de la tige interne pousse à la déviation d'une partie distale de la tige externe.

4. Cathéter selon la revendication 1, dans lequel la tige externe est conçue pour tourner autour de l'axe longitudinal par rapport à la tige interne, ou pour se déplacer le long de l'axe longitudinal par rapport à la tige interne.

5. Cathéter selon la revendication 1, comprenant en outre un élément de commande supplémentaire (114) fixé à la tige externe et conçu pour commander (i) la rotation autour de l'axe longitudinal de la tige externe par rapport à la tige interne, (ii) la translation le long de l'axe longitudinal par rapport à la tige interne, ou (iii) la commande de la rotation autour de l'axe longitudinal de la tige externe et la translation de la tige externe par rapport à la tige interne.

6. Cathéter selon la revendication 1, comprenant en outre un indicateur (115) de la position de rotation ou de translation de la tige externe, éventuellement dans lequel l'indicateur comporte indicateur tactile ou visuel.

7. Cathéter selon la revendication 6, dans lequel l'élément de commande supplémentaire est distal par rapport au premier élément de commande.

8. Cathéter selon la revendication 1, dans lequel la tige interne comprend une lumière hors axe et le premier élément de traction s'étend à travers la lumière hors axe.

9. Cathéter selon la revendication 2, dans lequel la tige interne comprend au moins des première et seconde lumières diamétralement opposées, et le premier élément de traction s'étend à travers la première lumière diamétralement opposée et le second élément de traction s'étend à travers la seconde lumière diamétralement opposée.

10. Cathéter selon la revendication 1, l'effecteur comporte une structure planaire conçue pour supporter un réseau d'électrodes.

11. Cathéter selon la revendication 1, dans lequel la rotation autour de l'axe longitudinal de la tige externe est comprise entre environ 0 et 360 degrés, ou la rotation autour de l'axe longitudinal de la tige externe par rapport à la tige interne est comprise entre environ 0 et 90 degrés.

12. Cathéter selon la revendication 1, comprenant en outre des éléments d'arrêt (160, 162) conçus pour limiter la translation le long de l'axe longitudinal de la tige externe par rapport à la tige interne à une distance prédéterminée.

13. Cathéter selon la revendication 12, dans lequel les éléments d'arrêt comprennent un élément femelle et un élément mâle, l'élément femelle conçu avec des arrêts proximaux et distaux définissant une région de translation entre eux avec une longueur prédéterminée parallèle à l'axe longitudinal, l'élément mâle conçu pour se déplacer dans la région de translation entre les arrêts proximaux et distaux par rapport à l'élément femelle.

14. Cathéter selon la revendication 13, dans lequel l'un parmi les éléments femelle et mâle est disposé sur l'une parmi la surface intérieure de la tige externe et de la surface extérieure de la tige interne, et l'autre parmi les éléments femelle et mâle est disposé sur l'autre de la surface intérieure de la tige externe et de la surface extérieure de la tige interne.

15. Cathéter selon la revendication 14, dans lequel au moins l'un parmi les éléments mâle et femelle s'étend circonférentiellement sur la surface intérieure de la tige externe et sur la surface extérieure de la tige interne.

16. Cathéter selon la revendication 15, dans lequel l'autre parmi les éléments femelle et mâle (i) s'étend à partir de l'autre de la surface intérieure de la tige externe et de la surface extérieure de la tige interne, (ii) s'étend circonférentiellement sur l'autre parmi la surface intérieure de l'autre tige et l'autre surface de la tige interne, ou (iii) est conçu comme un corps avec une dimension radiale.

17. Cathéter selon la revendication 12, dans lequel la distance prédéterminée est comprise entre environ 0 et 1,0".
